# EUROPEAN PATENT SPECIFICATION

(11) **EP 1 218 410 B1**
(45) Date of publication and mention of the grant of the patent: **15.06.2005**
(21) Application number: 99949462.8
(22) Date of filing: 06.10.1999
(51) Int. Cl.: C07K 14/495, C07K 14/65, A23J 1/20

(54) **PROCESS FOR OBTAINING GROWTH FACTOR PREPARATIONS (TGF-BETA AND IGF-1) FROM MILK PRODUCTS HAVING LOW MUTUAL CROSS-CONTAMINATION**
VERFAHREN ZUR BEREITSTELLUNG VON WACHSTUMSFAKTOZUBEREITUNGEN (TGF-BETA UND IGF-1) MIT NIEDRIGER GEGENSEITIGER VERUNREINIGUNG AUS MILCHPRODUKTEN
PROCEDE POUR OBTENIR DES PREPARATIONS DE FACTEURS DE CROISSANCE (TGF-BETA ET IGF-1) A PARTIR DE PRODUITS LAITIERS AVEC UNE FAIBLE CONTAMINATION MUTUELLE CROISEE

(43) Date of publication of application: 03.07.2002
(73) Proprietor: Campina B.V., 5300 CC Zaltbommel (NL); N.V. Nutricia, 2700 MA Zoetermeer (NL)
(72) Inventor: KIVITS, Marinus, Gerardus , Cornelis, NL-5482 CC Schijndel (NL); HENDRICKS, Andor, Wilhelm, Joseph, NL-6031 GG Nederweert (NL); MALLEE, Leonard, Franciscus, NL-3581 WC Utrecht (NL)
(74) Representative: van Westenbrugge, Andries
(86) International application number: PCT/NL1999/000621
(87) International publication number: WO 2001/025276

(56) References cited:
- EP-A- 0 335 554
- EP-A- 0 556 083
- EP-A- 0 869 134
- WO-A-92/00014
- WO-A-92/00994
- BELFORD D.A. ET AL.: "Platelet derived growth factor, insulin-like growth factors, fibroplast growth factors and transforming growth factor beta do not account the cell growth activity present in bovine milk" JOURNAL OF ENDORINOLOGY, vol. 154, no. 1, 1997, page 45-55 XP000913489
- DATABASE WPI Section Ch, Week 199444 Derwent Publications Ltd., London, GB; Class B04, AN 1994-354660 XP002139233 & JP 06 279312 A (SNOW BRAND MILK PROD CO LTD), 4 October 1994 (1994-10-04)
- ROGERS ET AL: "Transforming growth factor beta in bovine milk: concentration, stability and molecular mass forms" JOURNAL OF ENDOCRINOLOGY, vol. 151, no. 1, 1996, page 77-86 XP000911289

## Description

The present invention relates to a process for obtaining a fraction comprising transforming growth factor β (TGF-β) in substantial absence of insulin-like growth factor (IGF-1) and a fraction comprising IGF-1 in substantial absence of TGF-β from milk products (milk or whey).

It has been known for some time that milk products contain growth factors that can have a beneficial activity. These growth factors are present in very low concentrations in the milk product, which is why they are sometimes referred to as micronutrients. They can be characterised by their isoelectric point, which is relatively high compared to other milk proteins and their molecular weight. The present invention in particular concerns the growth factors TGF-β and IGF-1.

TGF-β is a multifunctional protein found in all mammalian tissues. Currently, five forms of TGF-β are known, β1 to β5. It has been implicated in the development, differentiation and growth of tissue and the control of immune system function and carcinogenesis TGF-β can be isolated from natural sources (e.g. blood platelets), mammalian milk or colostrum or can be produced by recombinant cells.

IGF-1, an anabolic, i.e. growth promoting, growth factor, is a small protein (molecular weight about 7800) which plays an important role in bone metabolism. It has been shown to stimulate growth of cells in culture. Animal growth is also stimulated in pituitary deficient, normal and catabolic states. Kidney function is also improved. It can be produced using recombinant DNA technology, solid phase peptide synthesis, by isolating it from blood serum or from mammalian milk, e.g. bovine or human milk.

As described above, it is known that IGF-1 and TGF-β can be extracted from milk products such as milk or whey. However, with the methods that have been applied up to now, using an economically feasible process without many purification steps, it was only possible to obtain a mixture of these growth factors. For some uses, more in particular certain therapeutical applications it has been found that it is preferred to use an IGF-1 rich fraction essentially free of TGF-β and a TGF-β fraction essentially free of IGF-1.

An example of such a therapeutical use is that described in a copending application in the name of the Applicants. This document describes the use of TGF-β for preparing a pharmaceutical composition for preventing damage of the intestinal mucosa as a result of chemotherapy or radiotherapy. In this case it has been found that IGF-1 interferes with the activity of TGF-β. According to this application it is therefore necessary to supply TGF-β in the substantial absence of IGF-1 to the patient. Up to now such relatively pure TGF-β was only available from recombinant DNA techniques or by an economically unfeasible process for the isolation from milk (multiple step isolation, US5221734). These products are rather expensive and would make the treatment mentioned above inaccessible for large groups of patients.

WO 9200994 and WO 9529933 describe processes for isolating a plurality of growth factors from milk or whey. As described above, it is not always desired to have a mixture of growth factors, because some growth factors can have a negative effect on the activity of other growth factors. WO 9529933 further has the disadvantage that an acidification is applied. This results in separation of the growth factors from the binding proteins and also inactivates lactoperoxidase. The binding factors help survive the growth factors during passage in the intestine, where digestive enzymes may degrade the growth factors resulting in (partial) loss of activity.

EP 489884, or its equivalent WO 9200014, describes a process for obtaining a mixture of growth factors from colostrum by cationic exchange chromatography followed by adsorption chromatography on hydroxyapatite, recovering the fraction retained on the hydroxyapatite. It is described that by this method more than 50 % of all the growth factors is isolated. This document only refers to a mixture of growth factors and gives no clue to how the much higher level of immunoglobulins and the virtual absence of lactoperoxidase, as compared to milk and/or whey, influence the amount and the mutual contamination of IGF-1 and TGF-β in enriched growth factor preparations. Moreover, this document does not clarify whether the growth factors are still bound to binding factors.

EP 335554 relates to a cosmetic or pharmaceutical composition for topical application to mammalian skin or hair, said composition comprising a growth factor chosen from TGF-α, TGF-β andIGF-1, or fragments or mixtures thereof.

D.A. Belford et al. "*Platelet-derived growth factor, insulin-like growth factors, fibroblast growth factors and transforming growth factor* β *do not account for the cell growth activity present in bovine milk*", J. Endocrinology (1997) 154, 45-55 teach the use.of cation exchange chromatography to isolate a fraction comprising lactoperoxidase, immunoglobulines and growth factors from cheese whey.

EP 556083 discloses the separation of lactoperoxidase, a secretory component and lactoferrin from milk and related raw materials by using a cation exchanger.

US 5221734 describes a process to isolate a Milk Growth Factor (MGF) from milk or whey. This process requires many steps, including ionic exchange chromatography (IEC), hydrophobic interaction chromatography (HIC) and size exclusion chromatography, resulting in low yields of TGF-β. This makes this process economically unfeasible.

WO 9526984 relates to a process that includes a step wherein the milk product is heated to denature the lactoperoxidase. Thereafter the lactoperoxidase is separated from the composition, which increases the efficiency of the final purification of the growth factors. However, it is preferable to separate native lactoperoxidase, for commercial application as a natural preservative. Furthermore, it is desirable to increase the specific activity of the lactoperoxidase remaining after isolation of the growth factors.

It is an object of the present invention to provide a process for isolating TGF-β and IGF-1 from a milk product as relatively pure fractions (i.e. high proportion of one growth factor relative to the other growth factor) achieving a high yield of growth factors. It is a further object of the invention to provide these growth factors in a form which is suitable for oral administration. It is a further object of the invention to recover TGF-β and IGF-1 from milk products as relatively pure fractions and simultaneously recover native lactoperoxidase in a high yield.

According to the present invention, a process has been found to separate fractions rich in growth factors and containing binding factors, and at the same time produce a lactoperoxidase fraction with a high activity. The present invention relates to a process for extracting transforming growth factor β (TGF-β) and insulin-like growth factor I (IGF-1) from a milk product, comprising the steps of
a) recovering a basic fraction from the milk product by means of cationic exchange chromatography;
b) passing the fraction obtained in step a) over a hydroxyapatite column;
c) eluting the hydroxyapatite column sequentially with at least two eluents of increasing salt concentration, said eluents being selected from phosphate buffers, sodium chloride solutions and potassium chloride solutions, wherein the first eluent has a pH of 5.5 to 7 and a salt concentration of 0.05 to 0.2 M and the second eluent has a pH of 5.5 to 7 and a salt concentration of 0.2 to 0.3 M, to obtain two separate fractions:
   i) a fraction comprising IGF-1, wherein the ratio IGF-1 to TGF-β is greater than 10;
   ii) a fraction comprising TGF-β, wherein the ratio TGF-β to IGF-1 is greater than 5.

These steps can be followed by a further elution step d) wherein the hydroxyapatite column is eluted with an eluent having increased salt content or pH as compared to the eluents used in step c), said eluent being selected from phosphate buffers, sodium chloride solutions and potassium chloride solutions, in such a way as to obtain
iii) a fraction comprising lactoperoxidase.

The milk product which is used as a starting material for the present invention can be any mammalian milk or a milk derivative that contains growth factors, such as: cheese whey or casein whey. Preferably bovine milk or milk derivative is used. The milk can be subjected to a pretreatment such as mild pasteurization, and/or defattted using a centrifuge or a microfiltration step.

Preferably, the starting material is first subjected to a minimal heat treatment. This is advantageous because
1) in such a heat treatment a considerable proportion of the bacteria naturally occurring in milk are killed and
2) the denaturation of lactoperoxidase and other milk serum proteins is minimized.

A minimal heat treatment is understood to mean heating to 80 °C at the most, for not more than a few seconds.

Further, it is highly advantageous to strip the starting material of fat before subjecting it to the adsorption and elution steps. It has been found that after fat removal the column in which the cationic exchange resin is contained hardly becomes greased or clogged up during the step of adsorption to the cationic exchange resin. This prevents undue pressure build up in the column and unfavourable shortening of the adsorption cycles.

It is preferred to remove fat by microfiltration because this effects at the same time the reduction of the microbial contamination of the starting material. In this connection, microfiltration is understood to mean filtration with a filter having openings between 0.1 and 10 µm.

The cationic exchange resin used in step a) can be of any suitable type known in the field. It is preferred to use a cationic exchange resin of a mean particle size in excess of 100 µm and of a sufficient mechanical strength to resist high pressures. This has the advantage that the cationic exchange resin is resistant to high liquid loads, while the binding capacity is maintained. This makes it possible to process large amounts of liquid in short time, which is required for an industrially applicable process. Examples of suitable cationic exchange resins are S-Ceramic Hyper D, SP-Toyopearl, SP-Sepharose FastFlow and SP-Sepharose Big Beads.

Preferably the cationic exchange resin is equilibrated by buffering with a phosphate buffer of a pH value of 5.5 to 7.5. Then the milk product is passed through a column with the cationic exchange resin, for instance by pumping, whereby microcomponents adsorb from the starting material onto the cationic exchange resin. To prevent microbial growth, these processes are normally carried out at a temperature of 4 to 7 °C. However, the viscosity at this temperature leads to an unacceptable pressure build-up. Therefore, the adsorption is preferably carried out at a temperature of 15 to 20 °C to lower the viscosity of the milk or milk derivative, whilst maintaining a relatively hygienic condition.

According to a preferred embodiment the starting material is pumped at a high surface velocity (more than 500 cm per hour) and at a high liquid load (100-600 bed volumes per hour) over a cationic exchange resin having a mean particle size of 100-300 µm, as described in US 5,596,082. According to this embodiment a process is realised which is highly favourable from an economic point of view, having outstanding industrial applicability.

After the adsorption step, it is preferred to rinse the cationic exchange resin column of any residual milk product (starting material) by washing with a salt (NaCl) solution buffered at a pH between 5.5 and 7.5 and having a salt concentration of 0.15 molar or less.

After adsorption of the desired components onto the ionic exchange resin, an elution step is carried out. Preferably the components are eluted with a salt solution buffered at a pH between 5.5 and 7.5, preferably at a pH of about 6.5. As the salt preferably sodium chloride or potassium chloride is used, but also other salts e.g. ammonium acetate can be used. This results in a fraction containing the desired TGF-β, IGF-1 and lactoperoxidase.

In step b) of the process the fraction obtained after ionic exchange chromatography is passed over a hydroxyapatite column. Hydroxyapatite is a crystallized tricalcium phosphate which is used as a substrate for the absorption of proteins. Industrially applicable hydroxyapatite resins are Macroprep Ceramic Hydroxyapatite from Biorad and HA Ultrogel from Biosepra. Hydroxyapatite has unique separation characteristics due to both phosphate and calcium that can act as ligands. Only recently, hydroxyapatite material that can be applied on production scale became available. It is now used in several production scale protein recovery/purification processes.

According to this step of the present invention the milk fraction obtained in step a) is passed through the hydroxyapatite column, for instance by pumping, whereby microcomponents adsorb from the starting material onto the hydroxyapatite. The adsorption is preferably carried out at a pH greater than 5.5 and a phosphate concentration of 5 to 100 mmole/l.

After the absorption step the hydroxyapatite column is eluted sequentially with suitable eluting liquids. Possible eluents are phosphate buffers, sodium chloride and potassium chloride solutions. For the different fractions these eluents must have an increasing salt concentration. It is also possible to apply an increasing pH gradient. Other possible eluents are known to the person skilled in the art. It is preferred that the overall concentration range of the salt solutions used is between 0.01 to 1.0 M.

According to the invention, to obtain an IGF-1 enriched fraction the column is typically eluted with a phosphate buffer having a pH of 5.5 to 7 and a phosphate concentration of 0.05 to 0.2 M, preferably a pH of 6.0 and a phosphate concentration of 0.15 M. To obtain a TGF-β enriched fraction the column is subsequently eluted with a phosphate buffer having a pH of 5.5 to 7 and a concentration of 0.2 to 0.3 M, preferably a pH of 6.0 and a concentration of 0.25 M.

Overall, the present process results in a recovery of both IGF-1 and TGF-β of about 25 to 50 % compared to the amounts present in the starting material.

In a preferred embodiment of the invention a further elution step is carried out to recover a lactoperoxidase fraction. According to this embodiment the hydroxyapatite column is eluted with a phosphate buffer having a pH of 5.5 to 8 and a phosphate concentration of 0.3 to 0.5 M, preferably a pH of 7 and a phosphate concentration of 0.5 M. This results in a native lactoperoxidase fraction with a high activity, which is an additional benefit of the present invention.

The fractions obtained according to the present invention can be separated further into their respective components by means of known methods. Examples of separation methods that can be used are ionic exchange chromatography, hydrophobic interaction chromatography and size exclusion chromatography.

The final products can be treated further by techniques known in the art, to remove salt therefrom and/or to concentrate them. For salt removal for instance ultrafiltration or gel filtration can be used. For concentrating the fractions can be lyophilised or spraydried.

The present invention also relates to the different fractions of growth factors obtained with the present process. The invention thus also comprises a product containing a TGF-β rich fraction essentially free of IGF-1, wherein the ratio TGF-β to IGF-1 is greater than 5, preferably greater than 50. This product in particular contains more than 200 µg TGF-β per gram protein and less than 40 µg IGF-I per gram protein, as determined by ELISA (Enzyme Linked Immune Sorbent Assay). Generally, these fraction will contain 2000 µg TGF-β per gram protein at the most.

The invention further comprises a product containing an IGF-1 rich fraction essentially free of TGF-β, wherein the ratio IGF-1 to TGF-β is greater than 10, preferably greater than 100. This product in particular contains more than 50 µg IGF-1 per gram protein, and less than 10 µg TGF-β per gram protein. Typically, such a product contains 500 µg IGF-1 per gram protein at the most.

As described before, when applying a final extraction step a product can be obtained containing lactoperoxidase having at least 1200 Units per mg, as determined with the ABTS method, essentially according to Shindler et al. (1976), European Journal of Biochemistry 65, 325-331.

The IGF-and TGF-fractions further contain about 30 to 50 % immunoglobulins on protein. Their main function is to interact with harmful micro-organisms such as bacteria. This prevents the micro-organism from entering the blood circulation system. This situation in particular occurs when the intestinal mucosa of the patient has been damaged as a result of treatment with chemotherapy.

The immunoglobulins can be isolated from milk of mammals which have been hyperimmunised against certain pathogens or they can be isolated from normal bovine milk or whey. With the present process, using normal cow's milk as a starting material, a preparation is obtained rich in immunoglobulins, comprising IgG and IgA. 30 to 50 % of the protein fraction consists of immunoglobulins of the type IgG and IgA.

The TGF-β and IGF-1 fractions obtained according to the invention contain binding factors which are released upon acidification. Thus the latent and active forms of both growth factors may be determined by e.g performing a growth factor specific ELISA in the presence or absence of an acid treatment of the sample, respectively. The binding factors fulfil a role in the modulation of the growth factor activity and may protect the growth factors during passage through the gastrointestinal tract

The IGF-and TGF-fractions obtained according to the invention can be used for several purposes, one of which is the use during chemotherapy and radiotherapy for treatment and/or prevention of damage to the intestinal mucosa.

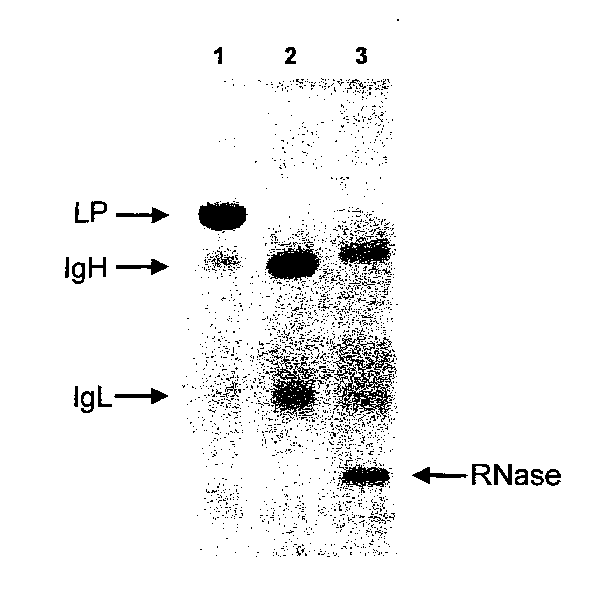

The present invention is further illustrated by means of the following examples and Figure 1 which shows the identification of immunoglobulins in an IGF-1 rich fraction. In the examples the following methods were used to analyse the products obtained.

Test kits for the determination of TGF-β and IGF-1 are commercially available. Test kit used:

Quantikine® for determination of human TGF-β from R&D Systems.

TGF-β is determined using a quantitative sandwich enzyme immunoassay technique (ELISA). A monoclonal antibody specific for human TGF-β2 has been pre-coated onto a microplate. Human and bovine TGF-β are identical so that the antibody will detect the bovine form. Standards and samples are pipetted into the wells and any TGF-β present is bound by the immobilized antibody. Prior to this step, since the TGF-β in milk is present in a latent form, it is first activated by an acid treatment to determine the total TGF-β concentration. This activation step is left out to determine the amount of active TGF-β.

After washing away any unbound substances, an enzyme-linked polyclonal antibody specific for TGF-β2 is added to the wells. Following a wash to remove any unbound antibody-enzyme reagent, a substrate solution is added to the wells and colour develops in proportion to the amount of TGF-β2 bound in the initial step. The colour development is stopped and the intensity of the colour measured.
TGF-β in samples is expressed as µg/g protein.

IGF-1: test kit used: IGF-1 ELISA DSL-10-2800 from Diagnostic Systems Laboratories, Inc.
IGF-1 is also determined by an enzymatically amplified "two-step"sandwich-type immunoassay similar to TGF-β. Samples, controls and prediluted unknowns are incubated in microtitration wells which have been coated with anti-IGF-1 antibody. IGF-1 in milk can be bound to binding proteins, and therefore, an activation step using acid similar to TGF-β is used when determining total IGF-1 concentration. The amount of free IGF-1 is determined when the activation step is left out.
IGF-1 in samples is expressed as µg/g protein protein.

### Protein

Protein in samples is determined with the Bradford method using Lactoferrin to make the standard curve.

### Example 1: Isolation of IGF-1, TGF-β and lactoperoxidase from milk

An ion exchange chromatography (IEC) column having a diameter of 10cm was packed with 1L of a strong cation exchanger (SP Sepharose Big Beads, Pharmacia). The column was preconditioned using a phosphate buffer (pH 6.5 0.025 M phosphate). The fat fraction of the milk was removed by means of centrifugation and 360L of the resulting skim milk was passed over the column at room temperature at a flow rate of 100BVH (Bed Volumes per Hour). The column was washed with 5L of a 0.10M NaCl pH6.5 solution. The adsorbed proteins were then fractionated by subsequently eluting the column with:
a) 5L of a 0.24M NaCl solution, pH6.5
b) 5L of a 1.00M NaCl solution, pH6.5

Fraction a) contains predominantly Lactoperoxidase and is rich in IGF-1 and TGF-β. Fraction b) is rich in angiogenin and lactoferrin. According to the results, fraction a) contains 9 g protein, including 7 g LP, 200µg IGF-1 and 1000µg TGF-β. Then the eluted fraction a) is diluted 20 fold and loaded onto a column containing 0.5L Hydroxyapatite (Biorad ceramic HAP type I, 40µm). at 15BVH. The column is washed with a buffer containing 60mM phosphate pH 6.0. The adsorbed proteins were then fractionated by subsequently eluting the column with:
c) 0.15M phosphate pH 6.0
d) 0.25M phosphate pH 6.0
e) 0.50M phosphate pH 7.0

Fraction c) contains 100µg IGF-1 (150µg /g protein) and is low in TGF-β (1µg TGF-β/g protein). Fraction d) contains 660µg TGF-β (1000µg/g protein) and is low in IGF-1 (5µg IGF-1/g protein). Fraction e) contains 7g LP (1200 Units/mg).

### Example 2: Isolation of IGF-1, TGF-β and lactoperoxidase from cheese whey

800 L microfiltered cheese whey were loaded onto 1L of SP Sepharose Big Beads at 150BVH. After washing the column with 5L of a 0.10M NaCl pH6.5 solution. The adsorbed proteins were fractionated by subsequently eluting the column with:
f) 5L of a 0.24M NaCl solution, pH6.5
g) 5L of a 1.00M NaCl solution, pH6.5

Fraction f) contains predominantly Lactoperoxidase and is rich in IGF-1 and TGF-β. Fraction g) is rich in angiogenin and lactoferrin. According to the results, fraction f) contains 8 g protein, including 6 g LP, 170µg IGF-1 and 150µg TGF-β. Then the eluted fraction is diluted 20 fold and charged onto a column containing 0.5L Hydroxyapatite (Biorad ceramic HAP type I, 40µm). at 15BVH. The column is washed with a buffer containing 60mM phosphate pH 6.0. The adsorbed proteins were then fractionated by subsequently eluting the column with:
h) 0.15M phosphate pH 6.0
i) 0.25M phosphate pH 6.0
j) 0.50M phosphate pH 7.0

Fraction h) contains 80µg IGF-1 (120µg /g protein) and is low in TGF-β (<1µg TGF-β/g protein). Fraction i) contains 100µg TGF-β (600µg/g protein) and is low in IGF-1 (8µg IGF-1/g protein). Fraction j) contains 6.5g LP (1200 Units/mg).

### Example 3: Isolation of IGF-1, TGF-β and lactoperoxidase from milk using different IEC elution conditions

The purity of the IEC fractions can be further increased by eluting the column under more stringent conditions.
Under identical conditions to those described in example 1, an IEC column was loaded with skim milk. The column was washed with a 5L of a 0.15M NaCl/10mM ammoniumacetate pH 5.5 solution. The growth factor rich fraction was then eluted by passing 3.5L of a 0.28M NaCl/10mM ammoniumacetate pH 5.5 solution over the column.
Although the yield of growth factors and lactoperoxidase in this step is slightly lower, the specific activity of the growth factors present in this fraction is higher versus the fraction obtained with the conditions as described in example 1, i.e. 40µg IGF/g protein and 180µg TGF/g protein.

### Example 4: Isolation of IGF-1, TGF-β and lactoperoxidase from milk using different hydroxyapatite elution conditions

The fractions bound on the hydroxyapatite column can also be separated using other elution conditions.
Under identical conditions to those described in example 1, the IEC eluate was loaded on the hydroxyapatite column and the hydroxyapatite column was washed with a buffer containing 0.12M NaCl/25mM phosphate pH7.0. The IGF-1 rich fraction was then eluted with a buffer containing 0.20M NaCl/25mM phosphate pH7.0 and thereafter the TGF-β rich fraction was obtained by eluting the column with a buffer containing 0.35M NaCl/25mM phosphate pH7.0. Then the lactoperoxidase was obtained by passing a solution containing 1M NaCl/25mM phosphate over the column.
The IGF-1 rich fraction contained 80µg IGF-1 (120µg/g protein) and is low in TGF-β (3µg TGF-β/g protein). The TGF-β rich fraction contained 500µg TGF-β (1100µg/g protein) and is
low in IGF-1 (1µg/g protein). According to this method 6.5g LP was obtained.

### Example 5: Identification of Immunoglobulins in IGF-1 rich fraction

The product resulting from Example 1 was evaluated by SDS Page to identify and quantify immunoglobulins (see figure 1). A 15% polyacrylamide gel was run under reducing and denaturing conditions using Phastsystem equipment (Pharmacia).
Lane 1: IEC fraction.
Lane 2: bovine IgG.
Lane 3: IGF-1 rich fraction. LP: Lactoperoxydase; IgH: heavy chain of IgG; IgL: light chain of IgG.

The protein band denoted RNase was identified by N-terminal sequencing.

From the figure it can be seen that the IGF-1 rich fraction in lane 3 does not contain any LP. Based on the color intensities of the bands, the immunoglobulin concentration in this sample is between 30 and 50%. The other major protein component was identified as RNase.

### Example 6 : Determination of latent and active forms of growth factors

Starting from milk , fractions were obtained after subsequent elutions over a cationic exchange and a hydroxyapatite column. These fractions were freezedried , solubilized in an appropriate buffer and then assayed with ELISA, essentially as described in the preceding text.
Part of the sample was used as is and part was acidified according to the testkit instructions. Protein in the samples was determined with the Bradford assay using lactoferrin as the calibration protein. The IGF-1 enriched fraction contained as is 75 microgram IGF-1/ g protein and after acidification 175 microgram IGF-1/ g protein. This means that 43% of the total IGF-1 activity is scored as free IGF-1 and 57% of the total IGF-1 activity is bound to binding proteins. By analogy, the TGF-β enriched fraction contained as is 7 microgram TGF-β/ g protein, whereas upon acidification 540 microgram TGF-β/ g protein was found. This demonstrates that almost 99% of TGF-β was present in the latent form.

## Claims

1. Process for extracting transforming growth factor β (TGF-β) and insulin-like growth factor 1 (IGF-1) from a milk product, comprising the steps of
a) recovering a basic fraction from the milk product by means of cationic exchange chromatography;
b) passing the fraction obtained in step a) over a hydroxyapatite column;
c) eluting the hydroxyapatite column sequentially with at least two eluents of increasing salt concentration, said eluents being selected from phosphate buffers; sodium chloride solutions and potassium chloride solutions, wherein the first eluent has a pH of 5.5 to 7 and a salt concentration of 0.05 to 0.2 M and the second eluent has a pH of 5.5 to 7 and a salt concentration of 0.2 to 0.3 M, to obtain two separate fractions:
i) a fraction comprising IGF-1, wherein the ratio IGF-1 to TGF-β is greater than 10;
ii) a fraction comprising TGF-β, wherein the ratio TGF-β to IGF-1 is greater than 5.

2. Process according to claim 1, further comprising the step of
d) eluting the hydroxyapatite column with an eluent having increased salt content or pH as compared to the eluents used in step c), said eluent being selected from phosphate buffers, sodium chloride solutions and potassium chloride solutions, to obtain
iii)a fraction comprising lactoperoxidase.

3. Process according to claim 2, wherein the eluent for obtaining fraction iii) has a pH of 5.5 to 8 and a salt concentration of 0.3 to 0.5 M.

4. Process according to any one of the preceding claims, wherein said eluents are phosphate buffers.

5. Process according to any one of the preceding claims, wherein step a) .is carried out by passing the milk product at a high surface velocity and a high liquid load through a column packed with the cationic exchange resin.

6. Process according to any one of the preceding claims, wherein the milk product is any mammalian milk, preferably milk from which fat has been removed.

7. Process according to claim 6, wherein the milk product is cheese whey.

8. Product obtainable by the process according to any of claims 1 to 7, which contains TGF-β in the substantial absence of IGF-1, wherein the ratio TGF-β to IGF-1 is greater than 5 and which contains 30 to 50 % immunoglobulins on protein.

9. Product according to claim 8, wherein the ratio TGF-β to IGF-1 is greater than 50.

10. Product according to claim 8 or 9, which contains more than 200 µg TGF-β per gram protein and less than 40 µg IGF-1 per gram protein.

11. Product obtainable by the process according to any of claims 1 to 7, which contains IGF-1 in the substantial absence of TGF-β, wherein the ratio IGF-1 to TGF-β is greater than 10 and which contains 30 to 50 % immunoglobulins on protein.

12. Product according to claim 11, wherein the ratio IGF-1 to TGF-β is greater than 100.

13. Product obtainable by the process according to any one of claims 1 to 7, which contains more than 50 µg IGF-1 per gram protein and less than 10 µg TGF-β per gram protein and which contains 30 to 50 % immunoglobulins on protein.

14. Product according to any of claims 8 to 13, containing binding factors for the growth factors, which can be released upon acidification.

## Patentansprüche

1. Verfahren zum Extrahieren des transformierenden Wachstumsfaktors β (TGF-β) und des Insulin-artigen Wachstumsfaktors 1 (IGF-1) aus einem Milchprodukt, das die Stufen umfasst:
a) Abtrennung einer basischen Fraktion aus dem Milchprodukt durch Kationenaustauschchromatographie;
b) Hindurchlaufenlassen der in der Stufe (a) erhaltenen Fraktion durch eine Hydroxyapatit-Kolonne;
c) sequentielles Eluieren der Hydroxyapatit-Kolonne mit mindestens zwei Eluierungsmitteln mit steigender Salzkonzentration, wobei die Eluierungsmittel ausgewählt werden aus Phosphatpuffern, Natriumchlorid-Lösungen und Kaliumchlorid-Lösungen, wobei das erste Eluierungsmittel einen pH-Wert von 5,5 bis 7 und eine Salzkonzentration von 0,05 bis 0,2 M und das zweite Eluierungsmittel einen pH-Wert von 5,5 bis 7 und eine Salzkonzentration von 0,2 bis 0,3 M aufweisen, zur Bildung von zwei getrennten Fraktionen:
i) einer Fraktion, die IGF-1 umfasst, in der das Verhältnis von IGF-1 zu TGF-β mehr als 10 beträgt; und
ii) einer Fraktion, die TGF-β umfasst, in der das Verhältnis von TGF-β zu IGF-1 mehr als 5 beträgt.

2. Verfahren nach Anspruch 1, das außerdem die Stufe umfasst:
d) Eluieren der Hydroxyapatit-Kolonne mit einem Eluierungsmittel, das im Vergleich zu den in der Stufe (c) verwendeten Eluierungsmitteln einen erhöhten Salzgehalt oder pH-Wert aufweist, wobei dieses Eluierungsmittel ausgewählt wird aus Phosphatpuffern, Natriumchlorid-Lösungen und Kaliumchlorid-Lösungen, zur Bildung
iii) einer Fraktion, die Lactoperoxidase umfasst.

3. Verfahren nach Anspruch 2, worin das Eluierungsmittel zur Bildung der Fraktion (iii) einen pH-Wert von 5,5 bis 8 und eine Salzkonzentration von 0,3 bis 0,5 M aufweist.

4. Verfahren nach einem der vorhergehenden Ansprüche, worin die Eluierungsmittel Phosphatpuffer sind.

5. Verfahren nach einem der vorhergehenden Ansprüche, worin die Stufe (a) durchgeführt wird durch Hindurchlaufenlassen des Milchprodukts mit einer hohen Oberflächengeschwindigkeit und einer hohen Flüssigkeitsbeladung durch eine Kolonne, die mit dem Kationenaustauscherharz gefüllt ist.

6. Verfahren nach einem der vorhergehenden Ansprüche, worin das Milchprodukt eine Säugetier-Milch, vorzugsweise eine Milch ist, aus der das Fett entfernt worden ist.

7. Verfahren nach Anspruch 6, worin das Milchprodukt Käsemolke ist.

8. Produkt, erhältlich nach dem Verfahren nach einem der Ansprüche1 bis 7, das TGF-β im Wesentlichen in Abwesenheit von IGF-1 enthält, wobei das Verhältnis von TGF-β zu IGF-1 mehr als 5 beträgt, und das 30 bis 50 % Immunglobuline, bezogen auf Protein, enthält.

9. Produkt nach Anspruch 8, worin das Verhältnis von TGF-β zu IGF-1 mehr als 50 beträgt.

10. Produkt nach Anspruch 8 oder 9, das mehr als 200 µg TGF-β pro Gramm Protein und weniger als 40 µg IGF-1 pro Gramm Protein enthält.

11. Produkt, erhältlich nach dem Verfahren nach einem der Ansprüche 1 bis 7, das IGF-1 im Wesentlichen in Abwesenheit von TGF-β enthält, wobei das Verhältnis von IGF-1 zu TGF-β mehr als 10 beträgt, und das 30 bis 50 % Immunglobuline, bezogen auf Protein, enthält.

12. Produkt nach Anspruch 11, worin das Verhältnis von IGF-1 zu TGF-β mehr als 100 beträgt.

13. Produkt, erhältlich nach dem Verfahren nach einem der Ansprüche 1 bis 7, das mehr als 50 µg IGF-1 pro Gramm Protein und weniger als 10 µg TGF-β pro Gramm Protein und 30 bis 50 % Immunglobuline, bezogen auf Protein, enthält.

14. Produkt nach einem der Ansprüche 8 bis 13, das Bindungsfaktoren für die Wachstumsfaktoren enthält, die beim Ansäuern freigesetzt werden können.

## Revendications

1. Procédé d'extraction du facteur de croissance transformant β (TGF-β) et du facteur de croissance 1 de type insuline (IGF-1) d'un produit laitier, comprenant les étapes consistant à :
a) recueillir une fraction basique à partir du produit laitier au moyen d'une chromatographie échangeuse de cations ;
b) faire passer la fraction obtenue dans l'étape a) sur une colonne d'hydroxyapatite ;
c) éluer séquentiellement la colonne d'hydroxyapatite avec au moins deux éluants selon une concentration en sel qui augmente, lesdits éluants étant choisis entre des tampons phosphatés, des solutions de chlorure de sodium et des solutions de chlorure de potassium, le premier éluant ayant un pH de 5,5 à 7 et une concentration en sel de 0,05 à 0,2 M, et le second éluant ayant un pH de 5,5 à 7 et une concentration en sel de 0,2 à 0,3 M, pour obtenir deux fractions distinctes :
i) une fraction comprenant l'IGF-1, dans laquelle le rapport de l'IGF-1 au TGF-β est supérieur à 10 ;
ii) une fraction comprenant le TGF-β, dans laquelle le rapport du TGF-β à l'IGF-1 est supérieur à 5.

2. Procédé selon la revendication 1, comprenant en outre l'étape consistant à :
d) éluer la colonne d'hydroxyapatite avec un éluant ayant une teneur en sel ou un pH accru, par comparaison avec les éluants utilisés dans l'étape c), ledit éluant étant choisi entre des tampons phosphatés, des solutions de chlorure de sodium et des solutions de chlorure de potassium, pour obtenir :
iii) une fraction comprenant une lactoperoxydase.

3. Procédé selon la revendication 2, dans lequel l'éluant permettant obtenir la fraction iii) a un pH de 5,5 à 8, et une concentration en sel de 0,3 à 0,5 M.

4. Procédé selon l'une quelconque des revendications précédentes, dans lequel lesdits éluants sont des tampons phosphatés.

5. Procédé selon l'une quelconque des revendications précédentes, dans lequel l'étape a) est conduite en faisant passer le produit laitier à une vitesse de déplacement élevée et une charge liquide élevée à travers une colonne garnie avec la résine échangeuse de cations.

6. Procédé selon l'une quelconque des revendications précédentes, dans lequel le produit laitier est n'importe quel lait de mammifère, de préférence un lait dont on a extrait les graisses.

7. Procédé selon la revendication 6, dans lequel le produit laitier est du lactosérum de fromage.

8. Produit que l'on peut obtenir par le procédé selon l'une quelconque des revendications 1 à 7, qui contient un TGF-β en l'absence substantielle d'IGF-1, dans lequel le rapport du TGF-β à l'IGF-1 est supérieur à 5, et qui contient 30 à 50 % d'immunoglobulines sur la protéine.

9. Produit selon la revendication 8, dans lequel le rapport du TGF-β à l'IGF-1 est supérieur à 50.

10. Produit selon la revendication 8 ou 9, qui contient plus de 200 µg de TGF-β par gramme de protéine et moins de 40 µg d'IGF-1 par gramme de protéine.

11. Produit que l'on peut obtenir par le procédé selon l'une quelconque des revendications 1 à 7, qui contient de l'IGF-1 en l'absence substantielle de TGF-β, dans lequel le rapport de l'IGF-1 au TGF-β est supérieur à 10, et qui contient 30 à 50 % d'immunoglobulines sur la protéine.

12. Produit selon la revendication 11, dans lequel le rapport de l'IGF-1 au TGF-β est supérieur à 100.

13. Produit que l'on peut obtenir par le procédé selon l'une quelconque des revendications 1 à 7, qui contient plus de 50 µg d'IGF-1 par gramme de protéine, et moins de 10 µg de TGF-β par gramme de protéine, et qui contient 30 à 50 % d'immunoglobulines sur la protéine.

14. Produit selon l'une quelconque des revendications 8 à 13, contenant des facteurs de liaison pour les facteurs de croissance, qui peuvent être libérés lors d'une acidification.
